# EUROPEAN PATENT SPECIFICATION

(11) **EP 4 093 261 B1**
(45) Date of publication and mention of the grant of the patent: **16.09.2026**
(21) Application number: 21763216.5
(22) Date of filing: 05.03.2021
(51) Int. Cl.: A61B 1/00

(54) **ENDOSCOPE HOOD**
ENDOSKOPHAUBE
CAPUCHON D'ENDOSCOPE

(30) Priority: 05.03.2020 AU 2020900679
(43) Date of publication of application: 30.11.2022
(73) Proprietor: Huelsen, Alexander, Brisbane QLD 4059 (AU)
(72) Inventor: Huelsen, Alexander, Brisbane QLD 4059 (AU)
(74) Representative: Murgitroyd & Company
(86) International application number: PCT/AU2021/050193
(87) International publication number: WO 2021/174311

(56) References cited:
- WO-A1-2014/123563
- WO-A1-2016/185358
- WO-A1-2017/068404
- WO-A2-2011/148172
- JP-A- 2006 192 087
- JP-A- 2008 178 511
- JP-A- 2015 213 635
- US-A1- 2009 012 362
- US-A1- 2018 168 437

## Description

### TECHNICAL FIELD

The present invention relates to an endoscope hood which may be attached to a distal end of an endoscope by press fitting.

### BACKGROUND

Any references to methods, apparatus or documents of the prior art are not to be taken as constituting any evidence or admission that they formed, or form part of the common general knowledge.

In endoscopic examinations/procedures, flexible instruments are used to view a body lumen, such as the gastrointestinal tract and many others. The instruments are provided with fiber optic or charge-couple device (CCD) cameras which enable images to be transmitted around bends and images to be produced to displays on a screen.

One problem is associated with the anatomy of the colon which consists of folds. As the tip of the endoscope passes along the lumen of the colon, these folds hamper the endoscope operator's ability to visualize the entire surface of the mucosa and in particular, detect pre-malignant and malignant lesions hidden within these folds. It is also difficult to maintain the position of the endoscope tip when a lesion or polyp is detected. It is hypothesized that this occurs because the endoscope tip is moving, the tip does not travel back at a constant speed but rather with jerks and slippages particularly when traversing a bend or length of colon where the bowel has been concertinaed over the endoscope shaft during intubation.

In view of the above, it is desirable to provide an improved endoscope cover or endoscope hood that addresses at least some of the issues of the prior art.

JP 2006 192087 discloses an endoscope hood comprising convex portions that are formed in a tapered shape.

### SUMMARY OF INVENTION

The invention provides an endoscope hood structured to be attached to a distal tip of an endoscope according to claim 1.

The endoscope hood comprises: a flexible and hollow cylindrical body extending between a first end and a second end with a lumen extending therethrough for receiving the distal tip of the endoscope through an opening at the first end of the cylindrical body and allowing the distal tip to be positioned adjacent the second end; the hollow cylindrical body comprising an internal surface for engaging and receiving said tip of the endoscope; and an outer surface of the cylindrical body is formed by profiled outer portions arranged in a plurality of rows between the first end and the second end of the cylindrical body wherein each of the profiled outer portions comprises respective contact surfaces that converges towards the second end of the cylindrical body such that the profiled outer portions provide low frictional resistance during forward movement of the endoscope hood covering the endoscope tip through a body cavity and a greater frictional resistance during a rearward movement of the endoscope hood covering the endoscope tip through the body cavity.

The plurality of rows of the profiled outer portions comprises a wedge with said contact surface of the wedge converging towards the second end of the cylindrical body whereby thickness of the wedge decreases in a direction towards the second end of the cylindrical body.

Each of the plurality of rows of the profiled outer portions comprises a plurality of said wedges circumferentially arranged along the outer portion of the cylindrical body.

Respective wedges for each of the plurality of rows of the profiled outer portions are adjacently arranged to form a column comprising of two or more of said wedges arranged between the first and second ends.

An edge portion for each wedge extends transversely across the outer portion of the cylindrical body, the edge portion being transverse relative to a central longitudinal axis passing through the enclosed hollow internal space of the body.

The endoscope hood further comprises:
a plurality of drainage apertures being provided adjacent the second end of the cylindrical body with; and
one or more said drainage channels provided along the outer portion of the body of the endoscope hood, each drainage channel corresponding to a respective drainage aperture.

The wedges in each said plurality of wedged arranged circumferentially are separated from each other a respective drainage channel.

In an embodiment, each drainage channel extends in a longitudinal direction from the second end towards the first end of the body.

In an embodiment, the endoscope hood further comprises a profiled stop member being positioned along the internal surface adjacent the second end of the hollow body to maintain a gap towards a forward viewing direction, the gap being between a lens of the endoscope positioned, during use, along the profiled stop member and an outer portion defining an opening at the second end of the hollow body wherein the profiled stop member comprises the plurality of drainage apertures to allow fluid to drain from an interior portion adjacent the second end of the endoscope hood to an external outer portion of the body of the endoscope hood.

In an embodiment, the profiled stop member is formed continuously with the outer portion defining the opening at the second end of the cylindrical body.

In an embodiment, the plurality of drainage apertures is circumferentially disposed along the profiled stop member of the cylindrical body.

In an embodiment, each of the drainage channels are circumferentially arranged along the outer portion of the body of the endoscope hood.

**In** an embodiment, the first end of the cylindrical body comprises flexible elements, each flexible element comprising a body portion which is substantially parallel to the cylindrical body in at least one operable configuration and a flared tip extending away from the first end and radially diverge away from the outer portion of the cylindrical body of the endoscope hood.

**In** an embodiment, the flexible elements are adapted to flex between:
a neutral position whereby at least some of the flexible elements are aligned along a longitudinal central axis of the body with the flared ends being divergent during a forward movement of the endoscope hood covering the endoscope tip through the body cavity; and
a divergent flared position for increased engagement with walls of a body cavity wherein during a rearward movement of the endoscope hood covering the endoscope tip through the body cavity the body portion of the flexible elements spread in a radially outwardly direction relative to the cylindrical body of the endoscope hood to engage with the walls of the body cavity.

**In** an embodiment, each of said flexible elements is integrally formed with the first end portion defining the opening at the first end of the hollow cylindrical body of the endoscope hood.

**In** another embodiment, each of said flexible elements is integrally formed with the internal surface of the cylindrical body to allow the flexible elements to extend from the first end of the cylindrical body.

**In** an embodiment, length of each of said flexible elements is less than or equal to height of the cylindrical body of the endoscope hood for preventing respective tip portions of the flexible elements from extending forwardly relative to the second end of the cylindrical body.

**In** an embodiment, the endoscope hood comprises at least a first plurality of said flexible elements and a second plurality of flexible elements wherein length of each of the flexible elements in the first plurality is greater than length of the flexible elements in the second plurality.

**In** an embodiment, the profiled stop member and the outer portion at the second end are structured such that the outer portion defining the opening at the second end lies in a plane that is inclined at an angle in the range of 1 and 10 degrees and more preferably less than 5 degrees relative to an imaginary plane of an outer portion defining the opening at the first end of the cylindrical body.

**In** an embodiment, during use, positioning the distal tip adjacent or in abutment with the stop member results in a camera of the distal tip being centered relative to the opening at the second end.

**In** another aspect, the endoscope hood comprises a plurality of drainage apertures preferably located along the profiled stop member to allow fluid to drain from an interior portion adjacent the second end of the endoscope hood to an external outer portion of the body of the endoscope hood.

**In** an embodiment, the plurality of drainage apertures are circumferentially disposed along the profiled stop portion of the cylindrical body.

**In** an embodiment, the endoscope hood further comprises one or more of the drainage channels provided along the outer portion of the body of the endoscope hood, each drainage channel corresponding to a respective drainage aperture.

**In** an embodiment, each drainage channel extends in a longitudinal direction from the second end towards the first end of the body.

**In** an embodiment, each of the drainage channels are circumferentially arranged along the outer portion of the body of the endoscope hood.

**In** another aspect, the invention provides an endoscope hood structured to be attached to a distal tip of an endoscope, the endoscope hood comprising: a flexible and hollow cylindrical body extending between a first end and a second end with a lumen extending therethrough for receiving the distal tip of the endoscope through an opening at the first end of the cylindrical body and allowing the distal tip to be positioned adjacent the second end; the hollow cylindrical body comprising an internal surface for engaging and receiving said tip of the endoscope; wherein the first end of the cylindrical body comprises flexible elements, each flexible element comprising a body portion which is substantially parallel to the cylindrical body in at least one operable configuration and a flared tip extending away from the first end and radially diverge away from the outer portion of the cylindrical body of the endoscope hood.

### BRIEF DESCRIPTION OF THE DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of the Invention in any way. The Detailed Description will make reference to a number of drawings as follows:
Figure 1 is a top perspective view of a "chip-on-tip" type of endoscope tip (prior art).
Figure 2 is a side view of the chip-on-tip type endoscope tip with an endoscope cap H (prior art).
Figure 3 is a first side view of an endoscope hood 100 in accordance with a first embodiment.
Figure 4 is a second side view of an endoscope hood 100.
Figure 5 is a third side view of an endoscope hood 100.
Figure 6 is a first frontal side perspective view of the endoscope hood 100.
Figure 7 is a second frontal side perspective view of the endoscope hood 100.
Figure 8 is a rear perspective view of the endoscope hood 100.
Figure 9 is a top perspective view of the endoscope hood 100.
Figure 10 is a first side view of an endoscope hood 200 in accordance with a second embodiment.
Figure 11 is a rear perspective view of the endoscope hood 200.
Figure 12 is a frontal perspective view of the endoscope hood 200.
Figure 13 is a side perspective view of an endoscope hood 300 in accordance with another embodiment.
Figure 14 is a rear perspective view of the endoscope hood 300.

### DETAILED DESCRIPTION OF PREFERRED EMBODIMENTS

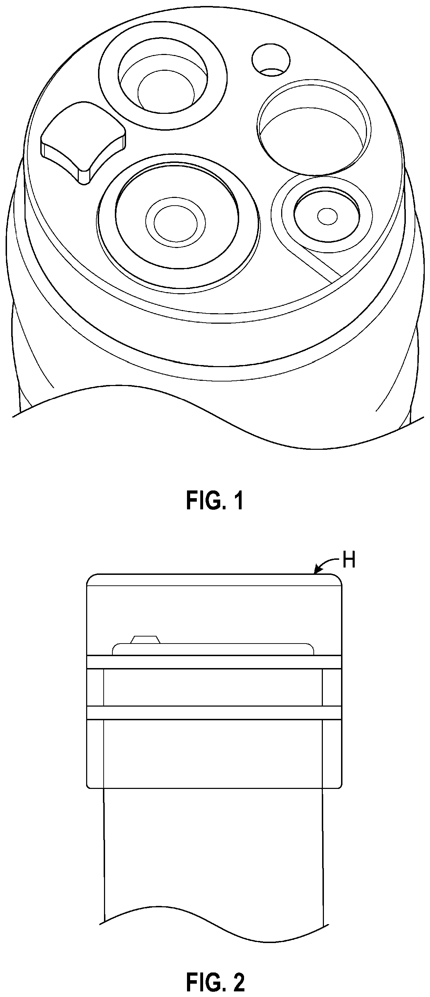

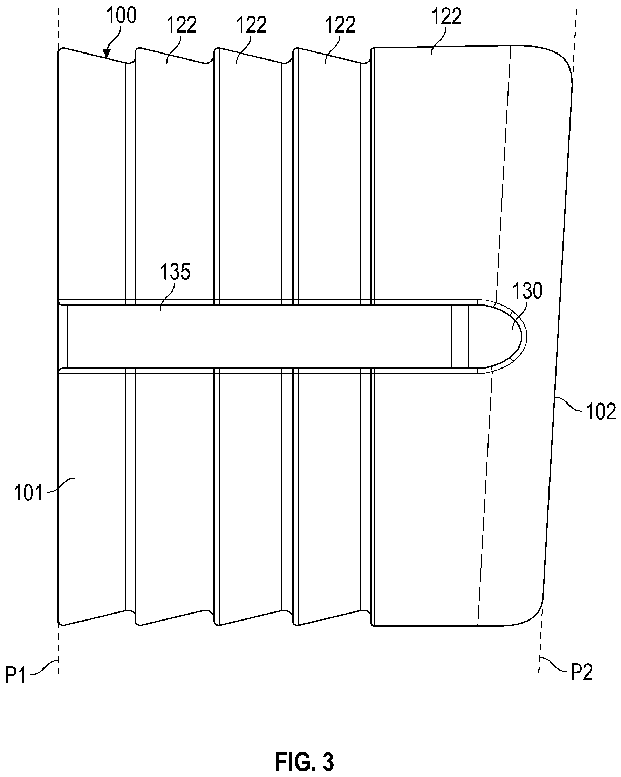

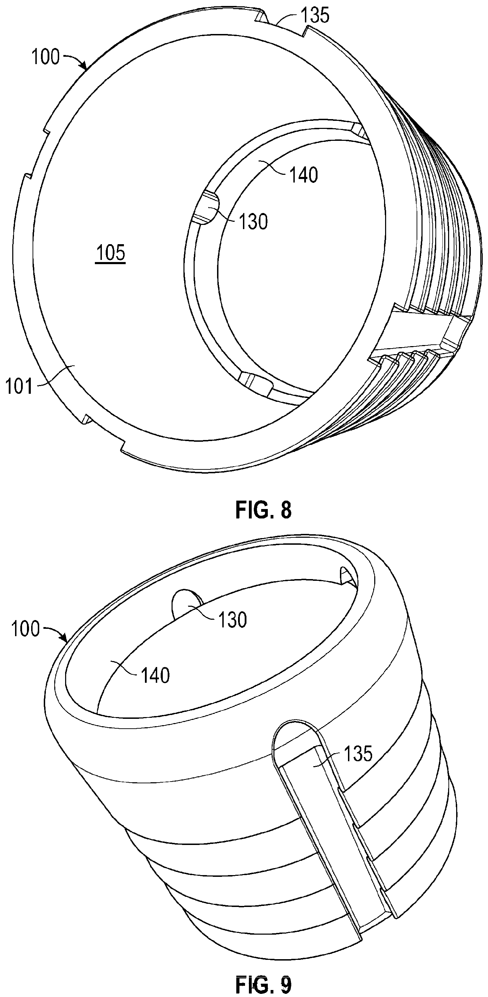

Figures 3 to 9 illustrate an endoscope hood 100 in accordance with a first embodiment of the present invention. As will be evident from the foregoing discussions, the endoscope hood 100 is suitable to be mounted onto a distal tip of an endoscope (shown in Figure 1) and inserted into a body cavity such as but not limited to the colon or other parts of the GI tract or other body lumens. The endoscope tip typically comprises one or more image capturing devices for viewing the body cavity and working lumens (such as for introducing tools to collect tissue samples, or for irrigation or suction, etc.), as is well known in the art. It is important to appreciate that the endoscope hood 100 is no way limited for use with any specific type of endoscope tip such as the tip shown in Figure 1.

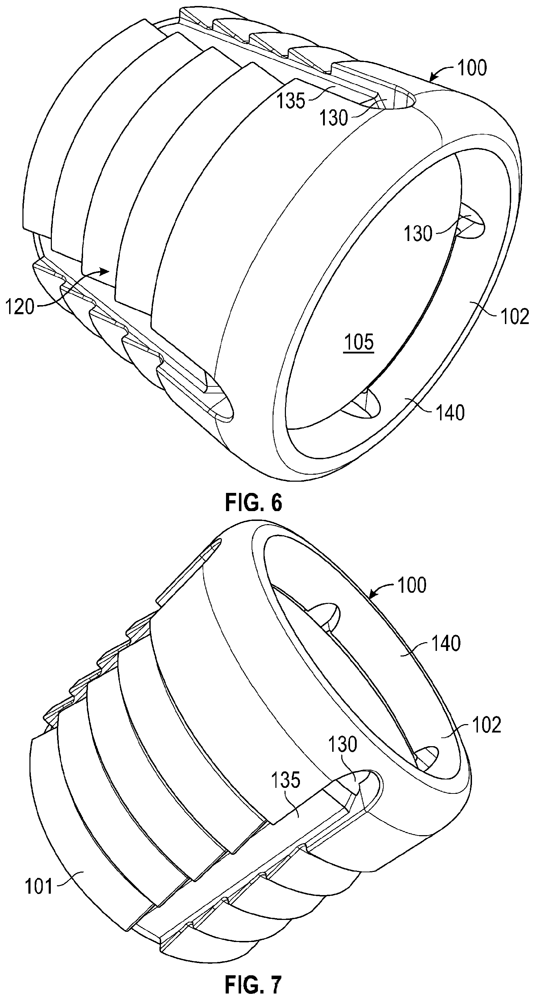

The endoscope hood 100 comprises a flexible and hollow cylindrical body 110 that extends between an insertion end 101 (also referred to as the "first end" throughout the specification) and a distal end 102 (also referred to as the "second end" throughout the specification). The hollow cylindrical body 110 comprises an internal surface 105 (See Figure 6) that defines an internal volume for receiving the distal tip of the endoscope. During use, the distal tip of the endoscope is press fitted into the insertion end 101 and passed through the lumen to position the distal tip adjacent the distal end 102 of the endoscope hood 100. The flexible material forming the cylindrical body 110 allows the internal surface 105 (See Figure 6) of the lumen to engage with the distal tip body of the endoscope and allow the cylindrical body 110 to be tightly fitted over the endoscope tip.

A profiled stop member 140 is positioned along the internal surface 105 of the cylindrical body. As shown clearly in Figures 6 and 7, the profiled stop member 140 is located adjacent the distal end 102 of the cylindrical body 110 to maintain a predetermined gap in a forward viewing direction when the endoscope tip is positioned along the profiled stop member 140. The profiled stop member 140 comprises a thickness that is sufficiently high to prevent the endoscope tip from being inadvertently pushed beyond the recommended focal distance. During use, the outer rim defining the distal end 102 of the cylindrical body 110 contacts walls of the body cavity (within which the endoscope hood 100 mounted on the endoscope tip is inserted). Positioning the endoscope tip against the profiled stop member 140 ensures that endoscope tip remains in focus during times of operating the endoscope.

Referring to Figures 1 and 2, it is evident that endoscope tips that incorporate the "chip-on-tip" technology require the camera chip (also known as a sensor chip) to be located at an off-centre location on the tip. Typically, this results in the camera chip protruding outwardly at the off-centre location on the endoscope tip. This is a practical consideration because a working lumen and light sources also need to be provided on the tip. As a result, using a prior art endoscope cover or sleeve H (shown in Figure 2) can result in the camera chip being positioned too close to the walls of the body cavity. This issue is particularly prevalent during colonoscopy and endoscopy of any part of the GI tract in general and this has a negative influence on the examination including but not limited to slower endoscope insertion and other major disadvantages in the very precise inspection of mucosal lesions (polyps, malignancies and precursors of malignancies).

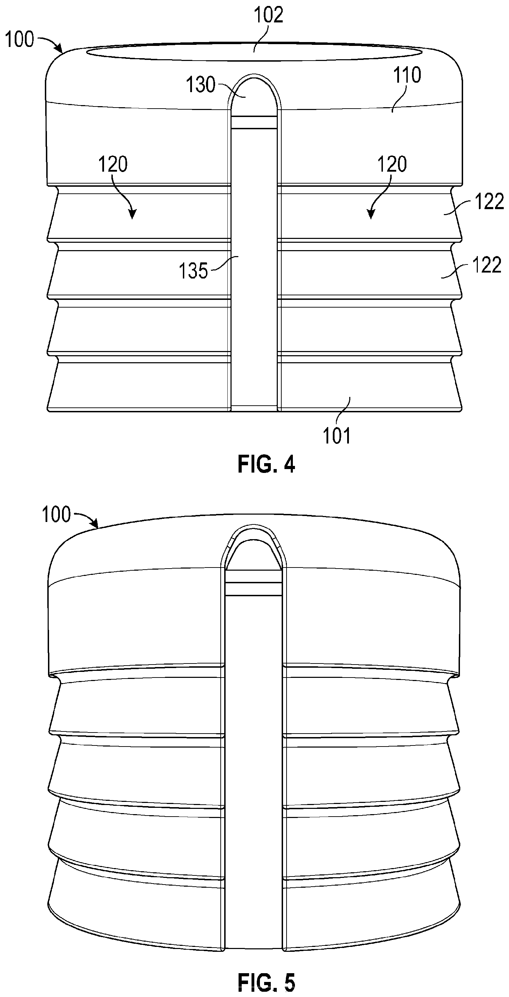

Most of the prior art endoscope caps or hoods that extend beyond the distal endoscope end are cut straight at the distal end. When such prior art caps are mounted on the chip-on-tip type of endoscope tips, the distal rim of the prior art endoscope caps is oriented at a slight angle relative to the plane of the camera in the endoscope tip (see Figure 2). As a result, any inspection of mucosal lesions using the prior art endoscope caps results in portions of the image being out of focus. The endoscope hood 100 addresses this issue by structuring the profiled stop member 140 and the outer portion or outer rim defining the second end 102 such that the outer rim at the second end 102 lies in an imaginary plane P2 (See Figure 3) that is slightly angled relative to an imaginary plane P1 (See Figure 3) of the outer rim defining the insertion opening at the insertion end 101 of the cylindrical body 110. The tip receiving edge of the profiled stop member 140 lies in a plane that is parallel to the imaginary plane P1. During use, the endoscope tip is positioned in abutment with the tip receiving edge of the stop member 140. Preferably, the plane of the outer rim P2 defining the second end 102 is angled at less than 10 degrees relative to the plane of the outer rim defining the first end 101. In the preferred embodiment, the profiled stop member 140 is formed continuously with the outer rim forming the second end 102 of the cylindrical body 110. As a result, the distance between the overall thickness of the profiled stop member 140 varies along the circumference of the cylindrical body. By way of example, minimum thickness of the profiled stop member 140 in at a first location may be 2mm and this thickness may vary along the circumference to result in a maximum thickness of say 3mm or even 4mm. As a result, outer wall height of the cylindrical body 110 also varies along the circumference. The variation in the wall height of the cylindrical body 110 is shown most clearly in Figures 4 and 5.

The profiled stop member 140 is also provided with a plurality of drainage apertures 130 that are circumferentially arranged along the profiled stop member 140. During use, mucosal fluid or other fluids can often accumulate within an interior hollow volume in the forward viewing direction (adjacent the second end 102) once the endoscope tip has been positioned in abutment with the profiled stop member 140. The drainage apertures 130 allow such accumulated fluids to drain out of the hollow volume. Each of the circumferentially arranged drainage aperture drains the fluid into a corresponding drainage channel 135 which is recessed into the outer wall of the cylindrical body 110 of the endoscope hood 100. Each channel 135 extends along the length of the outer wall towards the insertion end 101 of the cylindrical body 110 thereby directing the fluid away from the endoscope tip during use.

Each of the circumferential drainage channels 135 is separated by a column 120 of profiled wedge portions 122. The wedge portions 122 are shaped to provide low frictional resistance during forward movement of the endoscope hood 100 covering the endoscope tip through the body cavity and a greater frictional resistance during a rearward movement of the endoscope hood 100 covering the endoscope tip through the body cavity. Each wedge portion 122 includes a sloping contact surface that slopes from an edge portion (that extends transversely across the outer surface of the cylindrical body 110) of the wedge 122 towards the second end 102 in a radially inwardly direction. The orientation of the adjacently arranged wedge portions 122 in each column 120 lowers the frictional resistance between the wedge portions 122 and any walls of the body cavity (within which the endoscope hood 100 is being used). On the other hand, during rearward movement (typically extubation) the protruding edge portion of each wedge 122 assists with increasing frictional resistance with the walls of the body cavity which assists with flattening of the folds of body cavity.

Another important consideration relates to the combined effect of the drainage channels 135 with the transverse flow paths provided by the sloping surfaces of each wedge 122 which intersect with the drainage channels 135 and provide improved lateral drainage. This mechanism is therefore helpful in providing an overflow pathway that allows fluid to flow through lateral channels provided by the columns 120 of profiled wedges 122 provided along the outer body of the endoscope hood 100.

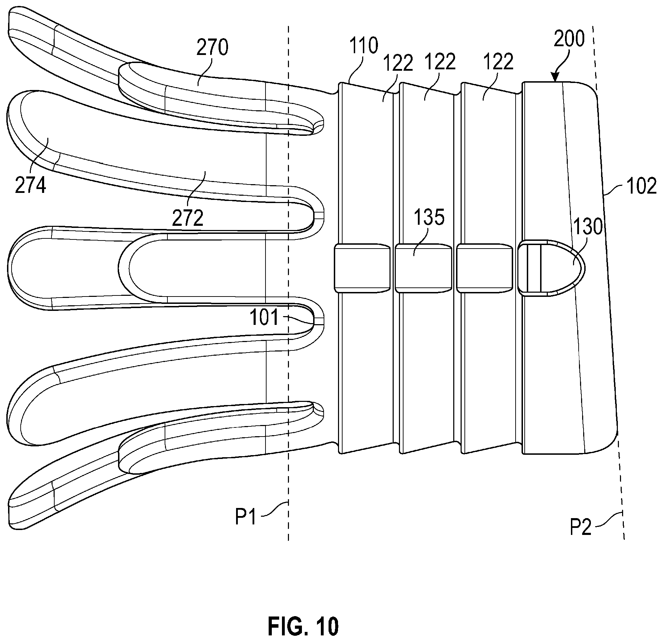

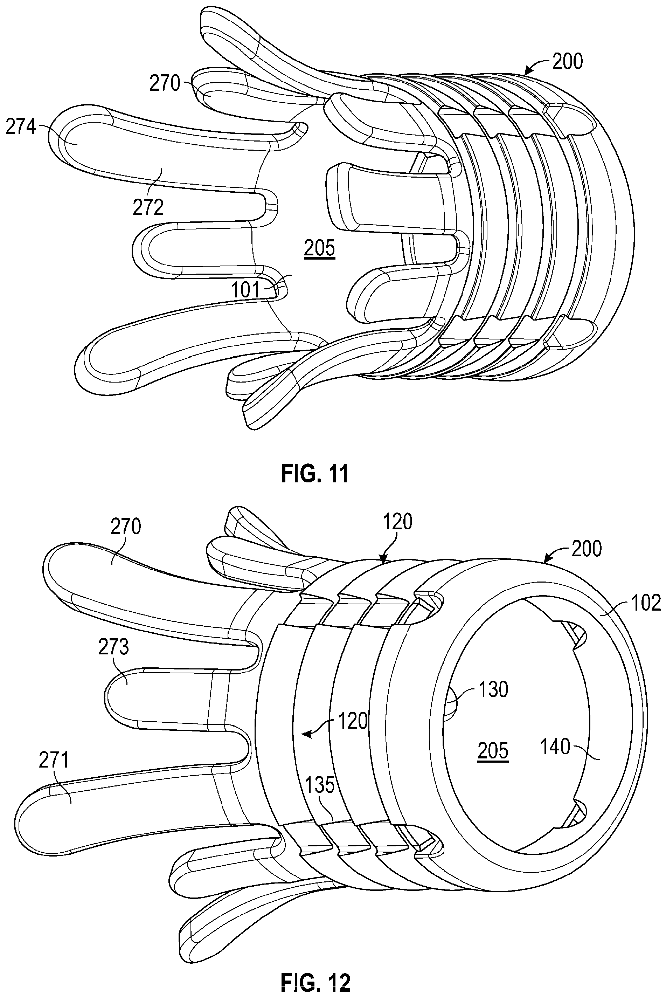

Referring to Figures 10 to 12, a second embodiment of the endoscope hood 200 has been shown. Like reference numerals represent like features that have been previously described. The endoscope hood 200 includes flexible elements 270 having a flared configuration that extend away from the insertion end 101 and radially diverge away from the outer portion of the cylindrical body 110 of the endoscope hood 200. Each of the elements 270 comprises a substantially elongate and flattened profile with flared tip portions 274 that are curved in a radially outwardly and divergent direction. Each of the elements 270 is integrally formed with the end portion of the cylindrical body 110 defining the insertion opening 101.

Each of the flexible elements 270 is adapted to flex between: a neutral position and a divergent flared position. In the neutral position, at least some of the flexible elements 270 are aligned along a longitudinal central axis of the body 110 during a forward movement of the endoscope hood 200 covering the endoscope tip through the body cavity. In the divergent flared position, for increased engagement with walls of a body cavity, rearward movement of the endoscope hood 200 covering the endoscope tip through the body cavity results in at least some of the flexible elements 270 to spread in a radially outwardly direction relative to the cylindrical body 110 of the endoscope hood 200 to engage with the walls of the body cavity. The filaments 270 are formed integrally with an outer surface 205 of the cylindrical body 110 It is important to note that each flexible element 270 comprises a straight body 272 with a permanently flared tip 274. Such a structure allows the tip 274 to remain divergent and engage walls of the body cavity (tom a limited extent) even during the forward movement of the endoscope tip whilst the body portion of the flexible element 270 remains substantially parallel to the cylindrical body 110.

The provision of the elements 270 assists the clinician in straightening the colon during insertion of the endoscope tip (with the mounted endoscope hood 200). During intubation of the gastrointestinal tract (colon, small bowel etc.) there are numerous bends in the colon and pushing forward through such bends usually stretches the bowel and therefore the endoscopes which use known endoscope hoods have to be pulled backwards. The provision of the flexible elements 270 alone and/or in combination with the profiled portions 122 prevents slipping of the endoscope hood and thereby allows the bowel length to be shortened behind the tip 274 of the flexible elements 270. The shape of the wedge shaped profile portions 122 continues to make intubation easier as discussed in the previous sections. Another advantage with the flexible elements 270 having naturally turned tips 274 is that during extubation, the flexible elements 270 diverge and fold smoothly along the turned portion of the tip 274 thereby making the movement of the endoscope hood 200 with the mounted endoscope smoother.

During withdrawal of the endoscope hood 200, for the flexible elements 270 to engage the colon walls during extubation, flexible elements 270 must be much longer. Therefore, provision of flexible elements with flared tip portion having varying lengths (specifically a first plurality of elements with a relatively longer length such as 271 and a second plurality of flexible elements 273) allows the endoscope hood 200 to extend the folds of the colon walls during intubation and extubation. It is also believed that providing flexible elements 270 with multiple lengths increases the overall resistance over a longer withdrawal distance. This is because, once a flexible element (say of a first length 271) has engaged the mucosal layer, it is likely to flex and fold towards the second end of the cylindrical body 110 thereby reducing the resistance offered. Having flexible elements 270 with varying lengths ensures that all the flexible elements 270do not fold back at the same time thereby increasing the overall resistance offered to the withdrawal movement of the endoscope hood 200.

Another important consideration relates the length of the flexible elements 270. The maximum length of the flexible elements 270 is chosen to ensure that during rearward movement, the flexible elements do not extend in a forwardly direction beyond the second end 102 of the cylindrical body. Such a feature avoids situations where the flexible elements 270 may obstruct or interfere with the endoscope tip camera's view in the forwardly direction.

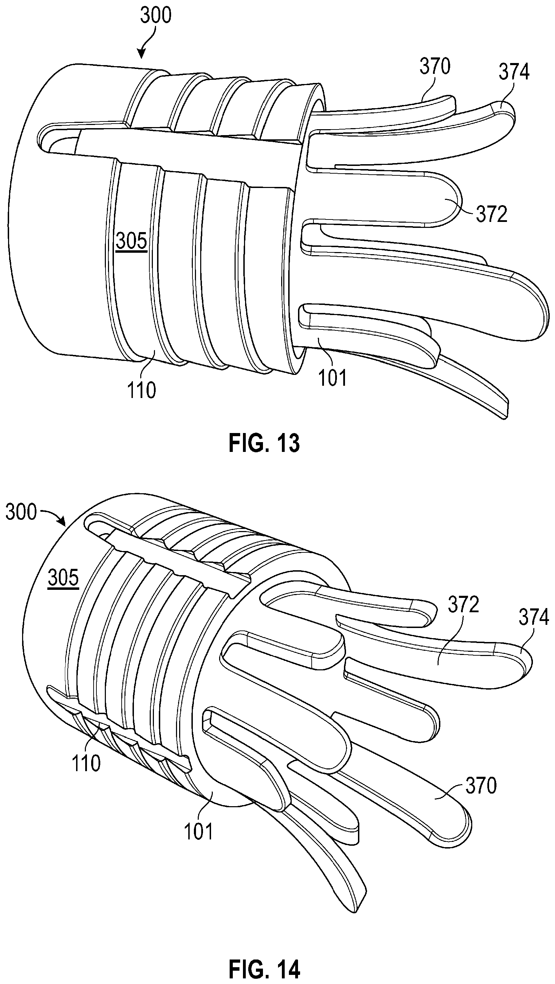

Referring to Figures 13 and 14, another embodiment of an endoscope hood 300 is illustrated. Once again, like reference numerals denote like features that have been previously illustrated. The endoscope hood 300 includes flexible elements 370 having a flared configuration that extend away from the insertion end 101 and radially diverge away from the outer portion of the cylindrical body 110 of the endoscope hood 300. Importantly, unlike the previously described endoscope hood 200, the flexible elements 370 are attached to and extend from an internal surface of the cylindrical body 110 of the endoscope hood 300 and do not extend from the outer surface 305 of the cylindrical body 110. Each of the elements 370 also comprises a substantially elongate and flattened profile with flared tip portions 374 that are curved in a radially outwardly and divergent direction. Each of the elements 370 is integrally formed with the inner surface of the cylindrical body 110 and is not directly attached with the outer surface 305 of the cylindrical body 110 of the endoscope hood 300.

The flexible elements 370 are also adapted to flex between: a neutral position and a divergent flared position. In the neutral position, at least some of the flexible elements 370 are aligned along a longitudinal central axis of the body 110 during a forward movement of the endoscope hood 300 covering the endoscope tip through the body cavity. In the divergent flared position, for increased engagement with walls of a body cavity, rearward movement of the endoscope hood 300 covering the endoscope tip through the body cavity results in at least some of the flexible elements 370 to spread in a radially outwardly direction relative to the cylindrical body 110 of the endoscope hood 300 to engage with the walls of the body cavity. It is important to note that each flexible element 370 comprises a straight body 372 with a permanently flared tip 374. Such a structure allows the tip 374 to remain divergent and engage walls of the body cavity (tom a limited extent) even during the forward movement of the endoscope tip whilst the body portion of the flexible element 370 remains substantially parallel to the cylindrical body 110.

The applicants hypothesize that in at least applications particularly in the divergent configuration, the flexible elements 370 are less likely to break away from the cylindrical body 110 of the endoscope hood 300 because the flexible elements 370 are attached to the inner surface of the cylindrical body 110.

The provision of the elements 370 assists the clinician in straightening the colon during insertion of the endoscope tip (with the mounted endoscope hood 300) in the same manner as described for the flexible elements 270 in the previous sections.

In compliance with the statute, the invention has been described in language more or less specific to structural or methodical features. The term "comprises" and its variations, such as "comprising" and "comprised of" is used throughout in an inclusive sense and not to the exclusion of any additional features.

It is to be understood that the invention is not limited to specific features shown or described since the means herein described comprises preferred forms of putting the invention into effect.

The invention is, therefore, claimed in any of its forms or modifications within the proper scope of the appended claims appropriately interpreted by those skilled in the art.

## Claims

1. An endoscope hood (100) structured to be attached to a distal tip of an endoscope, the endoscope hood comprising:
a flexible and hollow cylindrical body (110) extending between a first end (101) and a second end (102) with a lumen extending therethrough for receiving the distal tip of the endoscope through an opening at the first end (101) of the cylindrical body (110) and allowing the distal tip to be positioned adjacent the second end (102); the hollow cylindrical body (110) comprising an internal surface (105) for engaging and receiving said endoscope tip; and
an outer surface of the cylindrical body (110) is formed by profiled outer portions arranged in a plurality of rows between the first end (101) and the second end (102) of the cylindrical body wherein each of the plurality of rows of the profiled outer portions comprises respective contact surfaces that converges towards the second end (102) of the cylindrical body such that the profiled outer portions provide low frictional resistance during forward movement of the endoscope hood (100) covering the endoscope tip through a body cavity and a greater frictional resistance during a rearward movement of the endoscope hood (100) covering the endoscope tip through the body cavity;
wherein each of the plurality of rows of the profiled outer portions comprises a wedge (122) with said contact surface of the wedge (122) converging towards the second end (102) of the cylindrical body (110) whereby thickness of the wedge shaped profiled structure (122) decreases in a direction towards the second end (102) of the cylindrical body;
wherein each of the plurality of rows of the profiled outer portions comprises a plurality of said wedges (122) circumferentially arranged along the outer portion of the cylindrical body (110);
wherein respective wedges (122) for each of the plurality of rows of the profiled outer portions are adjacently arranged to form a column comprising of two or more of said wedges (122) arranged between the first (101) and second ends (102);
wherein an edge portion for each wedge (122) extends transversely across an outer portion of the cylindrical body (110), the edge portion being transverse relative to a central longitudinal axis passing through the enclosed hollow internal space of the cylindrical body; and
wherein the endoscope hood (100) further comprises:
a plurality of drainage apertures (130) being provided adjacent the second end (102) of the cylindrical body (110) with; and
one or more drainage channels (135) provided along the outer portion of the cylindrical body of the endoscope hood (100), each drainage channel (135) corresponding to a respective drainage aperture (130); and
wherein the wedges (122) in each said plurality of wedges (122) arranged circumferentially are separated from each other by a respective drainage channel (135).

2. An endoscope hood (100) in accordance with claim 1 wherein each drainage channel (135) extends in a longitudinal direction from the second end (102) towards the first end (101) of the cylindrical body.

3. An endoscope hood (100) in accordance with claim 1 or claim 2, further comprising a profiled stop member (140) being positioned along the internal surface (105) adjacent the second end (102) of the cylindrical body to maintain a gap towards a forward viewing direction, the gap being between a lens of the endoscope positioned, during use, along the profiled stop member and the outer portion defining an opening at the second end (102) of the cylindrical body (110) wherein the profiled stop member (140) comprises the plurality of drainage apertures (130) to allow fluid to drain from an interior portion adjacent the second end (102) of the endoscope hood (100) to an external outer portion of the cylindrical body of the endoscope hood (100).

4. An endoscope hood (100) in accordance with claim 3, wherein the profiled stop member (140) is formed continuously with the outer portion defining the opening at the second end (102) of the cylindrical body (102).

5. An endoscope hood (100) in accordance with any one of claims 3 or 4, wherein the plurality of drainage apertures (130) is circumferentially disposed along the profiled stop member (140) of the cylindrical body (110) and wherein each of the drainage channels (135) are circumferentially arranged along the outer portion of the cylindrical body of the endoscope hood (110).

6. An endoscope hood (100) in accordance with any one of the preceding claims, wherein the first end (101) of the cylindrical body (110) comprises flexible elements (270), each flexible element (270) comprising a body portion which is substantially parallel to the cylindrical body in at least one operable configuration and a flared tip extending away from the first end (101) and radially diverge away from the outer portion of the cylindrical body (110) of the endoscope hood (100) and wherein the flexible elements (270) are adapted to flex between:
a neutral position whereby at least some of the flexible elements (270) are aligned along a longitudinal central axis of the cylindrical body (110) with the flared tips being divergent during a forward movement of the endoscope hood (100) covering the endoscope tip through the body cavity; and
a divergent flared position for increased engagement with walls of a body cavity wherein during a rearward movement of the endoscope hood (100) covering the endoscope tip through the body cavity the body portion of the flexible elements (270) spread in a radially outwardly direction relative to the cylindrical body (110) of the endoscope hood (100) to engage with the walls of the body cavity.

7. An endoscope hood (100) in accordance with claim 6 wherein length of each of said flexible elements (270) is less than or equal to height of the cylindrical body (110) of the endoscope hood (100) for preventing respective tip portions of the flexible elements (270) from extending forwardly relative to the second end (102) of the cylindrical body (110).

8. An endoscope hood (100) in accordance with any one of claims 6 or 7 comprising at least a first plurality of said flexible elements (271) and a second plurality of flexible elements (273) wherein length of each of the flexible elements in the first plurality (271) is greater than length of the flexible elements in the second plurality (273).

9. An endoscope hood (100) in accordance with any one of claims 6 to 8 wherein each of said flexible elements (270, 271, 273) is integrally formed with the internal surface (105) of the cylindrical body (110) to allow the flexible elements (270, 271, 273) to extend from the first end (102) of the cylindrical body (110) and wherein each of said flexible elements (270, 271, 273) is integrally formed with the first end portion defining the opening at the first end (101) of the hollow cylindrical body (110) of the endoscope hood (100).

## Patentansprüche

1. Eine Endoskophaube (100), die strukturiert ist, um an einer distalen Spitze eines Endoskops angebracht zu werden, wobei die Endoskophaube Folgendes beinhaltet:
einen flexiblen und hohlen zylindrischen Körper (110), der sich zwischen einem ersten Ende (101) und einem zweiten Ende (102) erstreckt, mit einem sich dadurch erstreckenden Lumen, zum Aufnehmen der distalen Spitze des Endoskops durch eine Öffnung an dem ersten Ende (101) des zylindrischen Körpers (110) und zum Ermöglichen, dass die distale Spitze neben dem zweiten Ende (102) positioniert wird;
wobei der hohle zylindrische Körper (110) eine Innenfläche (105) zum Eingreifen in die Endoskopspitze und zum Aufnehmen derselben beinhaltet; und
eine Außenfläche des zylindrischen Körpers (110) durch profilierte Außenabschnitte gebildet ist, die in einer Vielzahl von Reihen zwischen dem ersten Ende (101) und dem zweiten Ende (102) des zylindrischen Körpers angeordnet sind, wobei jede der Vielzahl von Reihen der profilierten Außenabschnitte jeweilige Kontaktflächen beinhaltet, die zu dem zweiten Ende (102) des zylindrischen Körpers hin konvergieren, sodass die profilierten Außenabschnitte einen geringen Reibungswiderstand während einer Vorwärtsbewegung der Endoskophaube (100), die die Endoskopspitze abdeckt, durch einen Körperhohlraum und einen größeren Reibungswiderstand während einer Rückwärtsbewegung der Endoskophaube (100), die die Endoskopspitze abdeckt, durch den Körperhohlraum bereitstellen;
wobei jede der Vielzahl von Reihen der profilierten Außenabschnitte einen Keil (122) beinhaltet, wobei die Kontaktfläche des Keils (122) zu dem zweiten Ende (102) des zylindrischen Körpers (110) hin konvergiert, wodurch die Dicke der keilförmigen profilierten Struktur (122) in einer Richtung zu dem zweiten Ende (102) des zylindrischen Körpers hin abnimmt;
wobei jede der Vielzahl von Reihen der profilierten Außenabschnitte eine Vielzahl der Keile (122) beinhaltet, die umlaufend entlang des Außenabschnitts des zylindrischen Körpers (110) angeordnet sind;
wobei jeweilige Keile (122) für jede der Vielzahl von Reihen der profilierten Außenabschnitte nebeneinander angeordnet sind, um eine Säule zu bilden, die zwei oder mehr der Keile (122) beinhaltet, die zwischen dem ersten (101) und dem zweiten Ende (102) angeordnet sind;
wobei sich ein Kantenabschnitt für jeden Keil (122) quer über einen Außenabschnitt des zylindrischen Körpers (110) erstreckt, wobei der Kantenabschnitt quer zu einer zentralen Längsachse verläuft, die durch den umschlossenen hohlen Innenraum des zylindrischen Körpers verläuft; und
wobei die Endoskophaube (100) ferner Folgendes beinhaltet:
eine Vielzahl von Drainagedurchlässen (130), die neben dem zweiten Ende (102) des zylindrischen Körpers (110) bereitgestellt sind; und
einen oder mehrere Drainagekanäle (135), die entlang des Außenabschnitts des zylindrischen Körpers der Endoskophaube (100) bereitgestellt sind, wobei jeder Drainagekanal (135) einem jeweiligen Drainagedurchlass (130) entspricht; und
wobei die Keile (122) in jeder der Vielzahl von Keilen (122), die umlaufend angeordnet sind, durch einen jeweiligen Drainagekanal (135) voneinander getrennt sind.

2. Endoskophaube (100) gemäß Anspruch 1, wobei sich jeder Drainagekanal (135) in einer Längsrichtung von dem zweiten Ende (102) zu dem ersten Ende (101) des zylindrischen Körpers erstreckt.

3. Endoskophaube (100) gemäß Anspruch 1 oder Anspruch 2, ferner beinhaltend ein profiliertes Anschlagelement (140), das entlang der Innenfläche (105) neben dem zweiten Ende (102) des zylindrischen Körpers positioniert ist, um einen Spalt in einer Vorwärtsblickrichtung aufrechtzuerhalten, wobei der Spalt zwischen einer Linse des Endoskops, die während der Verwendung entlang des profilierten Anschlagelements positioniert ist, und dem Außenabschnitt, der eine Öffnung an dem zweiten Ende (102) des zylindrischen Körpers (110) definiert, liegt, wobei das profilierte Anschlagelement (140) die Vielzahl von Drainagedurchlässen (130) beinhaltet, um zu ermöglichen, dass Fluid von einem inneren Abschnitt neben dem zweiten Ende (102) der Endoskophaube (100) zu einem äußeren Außenabschnitt des zylindrischen Körpers der Endoskophaube (100) abfließt.

4. Endoskophaube (100) gemäß Anspruch 3, wobei das profilierte Anschlagelement (140) durchgehend mit dem Außenabschnitt gebildet ist, der die Öffnung an dem zweiten Ende (102) des zylindrischen Körpers (102) definiert.

5. Endoskophaube (100) gemäß einem der Ansprüche 3 oder 4, wobei die Vielzahl von Drainagedurchlässen (130) umlaufend entlang des profilierten Anschlagelements (140) des zylindrischen Körpers (110) eingerichtet ist und wobei jeder der Drainagekanäle (135) umlaufend entlang des Außenabschnitts des zylindrischen Körpers der Endoskophaube (110) angeordnet ist.

6. Endoskophaube (100) gemäß einem der vorhergehenden Ansprüche, wobei das erste Ende (101) des zylindrischen Körpers (110) flexible Elemente (270) beinhaltet, wobei jedes flexible Element (270) einen Körperabschnitt, der in mindestens einer betriebsfähigen Konfiguration im Wesentlichen parallel zu dem zylindrischen Körper ist, und eine aufgeweitete Spitze beinhaltet, die sich von dem ersten Ende (101) weg erstreckt und von dem Außenabschnitt des zylindrischen Körpers (110) der Endoskophaube (100) radial weg divergiert, und wobei die flexiblen Elemente (270) angepasst sind, um sich zwischen Folgendem zu verbiegen:
einer neutralen Position, wodurch mindestens einige der flexiblen Elemente (270) entlang einer zentralen Längsachse des zylindrischen Körpers (110) ausgerichtet sind,
wobei die aufgeweiteten Spitzen während einer Vorwärtsbewegung der Endoskophaube (100), die die Endoskopspitze abdeckt, durch den Körperhohlraum divergent sind; und
einer aufgeweiteten Divergenzposition für einen erhöhten Eingriff mit Wänden eines Körperhohlraums, wobei sich während einer Rückwärtsbewegung der Endoskophaube (100), die die Endoskopspitze abdeckt, durch den Körperhohlraum der Körperabschnitt der flexiblen Elemente (270) in einer radial nach außen gerichteten Richtung relativ zu dem zylindrischen Körper (110) der Endoskophaube (100) ausbreitet, um mit den Wänden des Körperhohlraums in Eingriff zu kommen.

7. Endoskophaube (100) gemäß Anspruch 6, wobei die Länge jedes der flexiblen Elemente (270) kleiner oder gleich der Höhe des zylindrischen Körpers (110) der Endoskophaube (100) ist, um zu verhindern, dass sich jeweilige Spitzenabschnitte der flexiblen Elemente (270) relativ zu dem zweiten Ende (102) des zylindrischen Körpers (110) nach vorne erstrecken.

8. Endoskophaube (100) gemäß einem der Ansprüche 6 oder 7, die mindestens eine erste Vielzahl von flexiblen Elementen (271) und eine zweite Vielzahl von flexiblen Elementen (273) beinhaltet, wobei die Länge jedes der flexiblen Elemente in der ersten Vielzahl (271) größer als die Länge der flexiblen Elemente in der zweiten Vielzahl (273) ist.

9. Endoskophaube (100) gemäß einem der Ansprüche 6 bis 8, wobei jedes der flexiblen Elemente (270, 271, 273) integral mit der Innenfläche (105) des zylindrischen Körpers (110) gebildet ist, um zu ermöglichen, dass sich die flexiblen Elemente (270, 271, 273) von dem ersten Ende (102) des zylindrischen Körpers (110) erstrecken, und wobei jedes der flexiblen Elemente (270, 271, 273) integral mit dem ersten Endabschnitt, der die Öffnung an dem ersten Ende (101) des hohlen zylindrischen Körpers (110) der Endoskophaube (100) definiert, gebildet ist.

## Revendications

1. Un capuchon d'endoscope (100) structuré pour être fixé à une pointe distale d'un endoscope, le capuchon d'endoscope comprenant :
un corps cylindrique (110) creux et flexible s'étendant entre une première extrémité (101) et une deuxième extrémité (102) avec une lumière s'étendant à travers celui-ci de sorte à recevoir la pointe distale de l'endoscope à travers une ouverture au niveau de la première extrémité (101) du corps cylindrique (110) et à permettre à la pointe distale d'être positionnée de façon adjacente à la deuxième extrémité (102) ; le corps cylindrique (110) creux comprenant une surface interne (105) de sorte à engager et à recevoir ladite pointe d'endoscope ; et
une surface externe du corps cylindrique (110) est formée par des portions externes profilées agencées en une pluralité de rangées entre la première extrémité (101) et la deuxième extrémité (102) du corps cylindrique où chaque rangée de la pluralité de rangées des portions externes profilées comprend des surfaces de contact respectives qui convergent vers la deuxième extrémité (102) du corps cylindrique de telle sorte que les portions externes profilées fournissent une faible résistance au frottement pendant un déplacement vers l'avant du capuchon d'endoscope (100) couvrant la pointe d'endoscope à travers une cavité corporelle et une résistance au frottement plus importante pendant un déplacement vers l'arrière du capuchon d'endoscope (100) couvrant la pointe d'endoscope à travers la cavité corporelle ;
où chaque rangée de la pluralité de rangées des portions externes profilées comprend un coin (122), ladite surface de contact du coin (122) convergeant vers la deuxième extrémité (102) du corps cylindrique (110) moyennant quoi l'épaisseur de la structure profilée en forme de coin (122) diminue dans une direction vers la deuxième extrémité (102) du corps cylindrique ;
où chaque rangée de la pluralité de rangées des portions externes profilées comprend une pluralité desdits coins (122) agencés de manière circonférentielle le long de la portion externe du corps cylindrique (110) ;
où des coins respectifs (122) pour chaque rangée de la pluralité de rangées des portions externes profilées sont agencés de manière adjacente afin de former une colonne comprenant deux desdits coins (122) ou plus agencés entre la première (101) et la deuxième extrémité (102) ;
où une portion de bord pour chaque coin (122) s'étend transversalement de part en part d'une portion externe du corps cylindrique (110), la portion de bord étant transversale par rapport à un axe longitudinal central passant à travers l'espace interne creux clos du corps cylindrique ; et
où le capuchon d'endoscope (100) comprend en outre :
une pluralité d'orifices de drainage (130) fournis de façon adjacente à la deuxième extrémité (102) du corps cylindrique (110) ; et
un ou plusieurs canaux de drainage (135) fournis le long de la portion externe du corps cylindrique du capuchon d'endoscope (100), chaque canal de drainage (135) correspondant à un orifice de drainage (130) respectif ; et
où les coins (122) dans chaque dite pluralité de coins (122) agencés de manière circonférentielle sont séparés les uns des autres par un canal de drainage respectif (135).

2. Un capuchon d'endoscope (100) conformément à la revendication 1 où chaque canal de drainage (135) s'étend dans une direction longitudinale depuis la deuxième extrémité (102) vers la première extrémité (101) du corps cylindrique.

3. Un capuchon d'endoscope (100) conformément à la revendication 1 ou à la revendication 2, comprenant en outre un organe d'arrêt profilé (140) qui est positionné le long de la surface interne (105) de façon adjacente à la deuxième extrémité (102) du corps cylindrique afin de maintenir un intervalle dans une direction de visualisation vers l'avant, l'intervalle se trouvant entre une lentille de l'endoscope positionnée, pendant l'utilisation, le long de l'organe d'arrêt profilé et la portion externe définissant une ouverture au niveau de la deuxième extrémité (102) du corps cylindrique (110) où l'organe d'arrêt profilé (140) comprend la pluralité d'orifices de drainage (130) afin de permettre au fluide d'être drainé depuis une portion intérieure adjacente à la deuxième extrémité (102) du capuchon d'endoscope (100) jusqu'à une portion externe extérieure du corps cylindrique du capuchon d'endoscope (100).

4. Un capuchon d'endoscope (100) conformément à la revendication 3, où l'organe d'arrêt profilé (140) est formé de manière continue avec la portion externe définissant l'ouverture au niveau de la deuxième extrémité (102) du corps cylindrique (102).

5. Un capuchon d'endoscope (100) conformément à l'une quelconque des revendications 3 ou 4, où la pluralité d'orifices de drainage (130) est disposée de manière circonférentielle le long de l'organe d'arrêt profilé (140) du corps cylindrique (110) et où chacun des canaux de drainage (135) est agencé de manière circonférentielle le long de la portion externe du corps cylindrique du capuchon d'endoscope (110).

6. Un capuchon d'endoscope (100) conformément à l'une quelconque des revendications précédentes, où la première extrémité (101) du corps cylindrique (110) comprend des éléments flexibles (270), chaque élément flexible (270) comprenant une portion de corps qui est substantiellement parallèle au corps cylindrique dans au moins une configuration apte à fonctionner et une pointe évasée s'étendant en s'éloignant de la première extrémité (101) et divergeant radialement en s'éloignant de la portion externe du corps cylindrique (110) du capuchon d'endoscope (100) et où les éléments flexibles (270) sont conçus pour fléchir entre :
une position neutre moyennant quoi au moins certains des éléments flexibles (270) sont alignés le long d'un axe central longitudinal du corps cylindrique (110), les pointes évasées étant divergentes pendant un déplacement vers l'avant du capuchon d'endoscope (100) couvrant la pointe d'endoscope à travers la cavité corporelle ; et
une position évasée divergente pour un engagement accru avec des parois d'une cavité corporelle où pendant un déplacement vers l'arrière du capuchon d'endoscope (100) couvrant la pointe d'endoscope à travers la cavité corporelle, la portion de corps des éléments flexibles (270) s'étale dans une direction radialement vers l'extérieur par rapport au corps cylindrique (110) du capuchon d'endoscope (100) afin de s'engager avec les parois de la cavité corporelle.

7. Un capuchon d'endoscope (100) conformément à la revendication 6 où la longueur de chacun desdits éléments flexibles (270) est inférieure ou égale à la hauteur du corps cylindrique (110) du capuchon d'endoscope (100) de sorte à empêcher des portions de pointe respectives des éléments flexibles (270) de s'étendre vers l'avant par rapport à la deuxième extrémité (102) du corps cylindrique (110).

8. Un capuchon d'endoscope (100) conformément à l'une quelconque des revendications 6 ou 7 comprenant au moins une première pluralité desdits éléments flexibles (271) et une deuxième pluralité d'éléments flexibles (273) où la longueur de chacun des éléments flexibles dans la première pluralité (271) est supérieure à la longueur des éléments flexibles dans la deuxième pluralité (273).

9. Un capuchon d'endoscope (100) conformément à l'une quelconque des revendications 6 à 8 où chacun desdits éléments flexibles (270, 271, 273) est formé d'un seul tenant avec la surface interne (105) du corps cylindrique (110) afin de permettre aux éléments flexibles (270, 271, 273) de s'étendre depuis la première extrémité (102) du corps cylindrique (110) et où chacun desdits éléments flexibles (270, 271, 273) est formé d'un seul tenant avec la première portion d'extrémité définissant l'ouverture au niveau de la première extrémité (101) du corps cylindrique (110) creux du capuchon d'endoscope (100).
